# EUROPEAN PATENT APPLICATION

(11) **EP 2 423 124 A1**
(43) Date of publication of application: **29.02.2012**
(21) Application number: 10767038.2
(22) Date of filing: 19.04.2010
(51) Int. Cl.: B65D 81/32, A61J 1/05, A61J 3/00

(54) **CONTAINER FOR MIXING BEFORE USE**

(30) Priority: 23.04.2009 JP 2009104780
(71) Applicant: Senju Pharmaceutical Co., Ltd., Osaka-shi, Osaka 541-0046 (JP); Hanshin Kasei Co., Ltd., Toyama-shi, Toyama 939-8183 (JP)
(72) Inventor: OTSUKA Tadashi, Kobe-shi Hyogo 651-2241 (JP); FUJIMOTO Takashi, Toyama-shi Toyama 939-8183 (JP)
(74) Representative: Knoop, Philipp
(86) International application number: PCT/JP2010/056937
(87) International publication number: WO 2010/122981

(57) **Abstract**

The present invention provides a before-use mixing container in which it is possible to perform a mixing operation for two types of medicinal solutions in its container independently of an uncovering operation for a cap or the like.

The before-use mixing container including a movable body 1 which has a tube section 11, a ceiled cylindrical inner tube 13 which is consecutively installed upright on an inner surface of the tube section, that partitions the tube section into two upper and lower chambers, and a first opening portion 13b drilled in a ceiling plane 13a of the inner tube, and in which a first medicinal solution A is contained in the lower chamber, an adapter 3 which has a fit-in tube section 31 which is tightly fit into the upper chamber of the movable body to be assembled so as to be rotatable with respect to the movable body, a partition wall surface 35 which is provided on an inner surface of the fit-in tube section, and is tightly attached to the ceiling plane of the inner tube of the movable body, and a second opening portion 35a which is drilled in the partition wall surface so as not to overlap the first opening portion, and a container main body 4 which has a lower mouth tube section 42 tightly fit into the upper chamber of the adapter, to be assembled so as not to be rotatable with respect to the adapter, and in which a second medicinal solution B is contained, in the before-use mixing container, when the movable body is relatively rotated with respect to the container main body, the first opening portion and the second opening portion are overlapped.

## Description

### [Technical Field]

The present invention relates to a before-use mixing container in which two types of medicinal solutions are stored in a separated state in one container, and these are mixed inside the container in use, and extracted for use.

### [Background Art]

There are many medicinal solutions such as eye drops, which are chemically unstable in a mixed state or a dissolved state, and in case of use of such a medicinal solution, it is necessary to perform a mixing operation immediately before use. There is a before-use mixing container as a container in which two types of medicinal solutions are stored in a separated state, and these are mixed in the container in use, and extracted for use.

As a before-use mixing container, for example, there has been known a container adopting a system that one medicinal solution and another medicinal solution are respectively contained in a first container body and a second container body in a separated state by use of isolation members in which one member having an opening portion and the other member having an opening portion are slidably fit into each other so that their opening portions do not face each other, and the both opening portions are made to face each other by a sliding operation of the isolation members, to mix the two types of medicinal solutions (refer to Patent Document 1).

### [Prior Art Document]

### [Patent Document]

[Patent Document 1] Japanese Patent No. 3228376 [Summary of the Invention]

### [Problems to be Solved by the Invention]

However, since an uncovering operation is accompanied at the time of mixing the two types of medicinal solutions in the before-use mixing container in Patent Document 1, there is, for example, a concern that the medicinal solutions may spill out when an uncovering operation is performed in a state in which the container is turned upside down by mistake. Accordingly, it is necessary to adopt amixingmethod independent of an uncovering operation in order to mix two types of medicinal solutions even when such an erroneous operation is performed.

The present invention has been achieved in consideration of the above-described circumstances. It is a primary object of the present invention to provide a before-use mixing container in which it is possible to perform a mixing operation for two types of medicinal solutions in the container independently of an uncovering operation for a cap or the like.

### [Means for Solving the Problem]

That is, the scope of the present invention is as follows.
[1] A before-use mixing container including
   a movable body which has a bottomed cylindrical tube section, a ceiled cylindrical inner tube which is consecutively installed upright on an inner side in a circumferential direction from an inner surface of the tube section, that partitions an internal space of the tube section into two upper and lower chambers, and a first opening portion drilled in a ceiling plane of the inner tube, and in which a first medicinal solution is contained in the lower chamber,
   an adapter which has a hollow cylindrical fit-in tube section which is tightly fit into the upper chamber of the movable body to be assembled so as to be rotatable with respect to the movable body, a partition wall surface which is provided on an inner surface of the fit-in tube section, and is tightly attached to the ceiling plane of the inner tube of the movable body in a state in which the partition wall is assembled to the movable body, and a second opening portion which is drilled in the partition wall surface so as not to overlap the first opening portion, and
   a container main body which has a lower mouth tube section which is tightly fit into the upper chamber surrounded by an upper surface of the partition wall surface of the adapter and the inner surface of the fit-in tube section, to be assembled so as not to be rotatable with respect to the adapter, and in which a second medicinal solution is contained, and in the container,
   when the movable body is relatively rotated with respect to the container main body, the first opening portion of the movable body and the second opening portion of the adapter are overlapped, and the container is capable of mixing the first medicinal solution and the second medicinal solution before use.
[2] In the before-use mixing container according to [1],
   the fit-in tube section of the adapter is provided with a large diameter section and a small diameter section having circular arc shapes in plan view, and in a state in which the fit-in tube section of the adapter is assembled to the upper chamber of the movable body, the large diameter section and the small diameter section of the fit-in tube section are respectively spaced from the inner surface of the tube section of the movable body to generate gaps, and
   a vertical line with a height exceeding a spaced distance between the inner surface of the tube section and the large diameter section, and with a height which does not exceed a spaced distance between the inner surface of the tube section and the small diameter section is provided on the inner surface of the tube section of the movable body, and the longitudinal vertical line faces the small diameter section.
[3] In the before-use mixing container according to [2], when the movable body is relatively rotated with respect to the container main body and the vertical line provided on the inner surface of the tube section of the movable body reaches a boundary portion between the small diameter section and the large diameter section of the fit-in tube section, the first opening portion and the second opening portion are overlapped.

### [Effects of the Invention]

According to the before-use mixing container of the present invention, since the container main body and the adapter are assembled so as not to be relatively rotatable, the adapter and the movable body are assembled so as to be relatively rotatable, and the two types of medicinal solutions are mixed by merely relatively rotating the movable body with respect to the container main body, it is possible to perform a mixing operation for the two types of medicinal solutions in the container independently of an uncovering operation for a cap or the like.

### [Brief Description of the Drawings]

Fig. 1 is an exploded perspective view of a before-use mixing container showing an embodiment of the present invention.
Fig. 2 is a cross-sectional view for explanation of the embodiment shown in Fig. 1.
Fig. 3A is a plan view of a movable body of Fig. 1, Fig. 3B is a cross-sectional view taken along line A-A thereof, and Fig. 3C is a bottom view thereof.
Fig. 4A is a plan view of an adapter of Fig. 1, Fig. 4B is a front view thereof, Fig. 4C is a bottom view thereof, and Fig. 4D is a cross-sectional view taken along line B-B thereof.
Fig. 5A is a front view of a container main body of Fig. 1, and Fig. 5B is a cross-sectional view taken along line C-C thereof.
Figs. 6 are views for explanation in a mixing operation. Fig. 6A is a view showing a state before the mixing operation, and Fig. 6B is a view showing a state after the mixing operation.

### [Embodiment for Carrying Out the Invention]

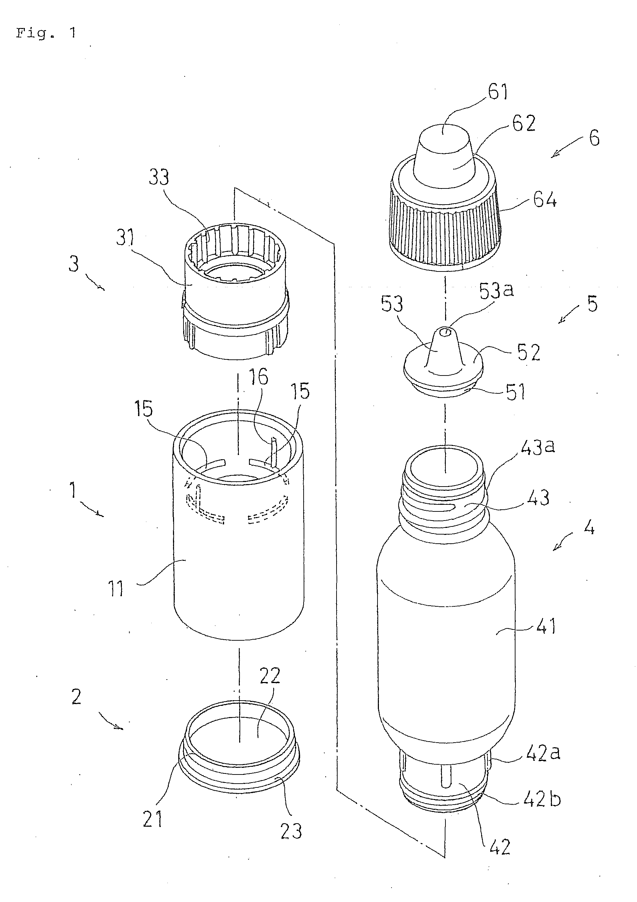

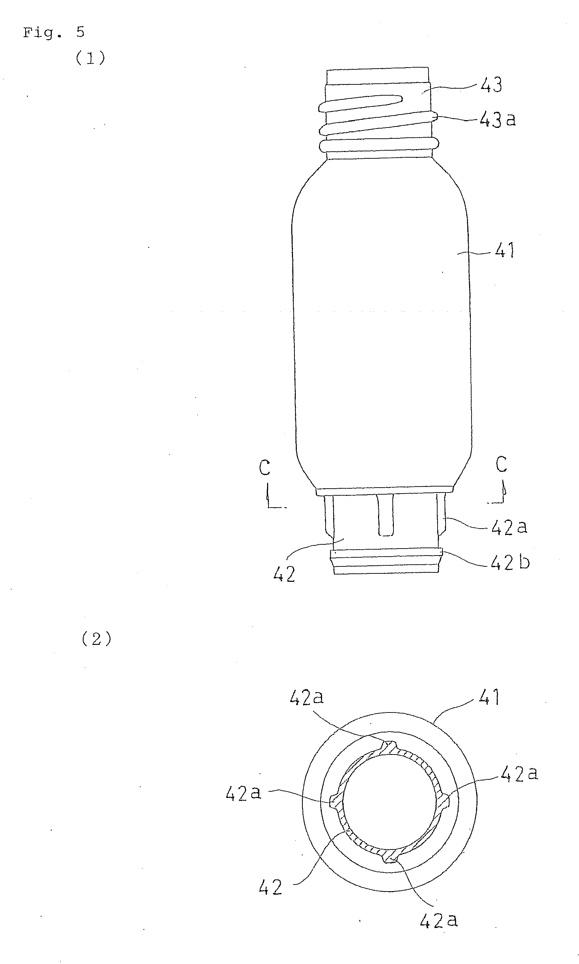

Hereinafter, am embodiment of the present invention will be described with reference to the drawings. Figs. 1 to 6 show the embodiment of the present invention. A before-use mixing container of the present invention is configured so that a bottom cover, a movable body, an adapter, a container main body, a nozzle, and a cap are integrally assembled.

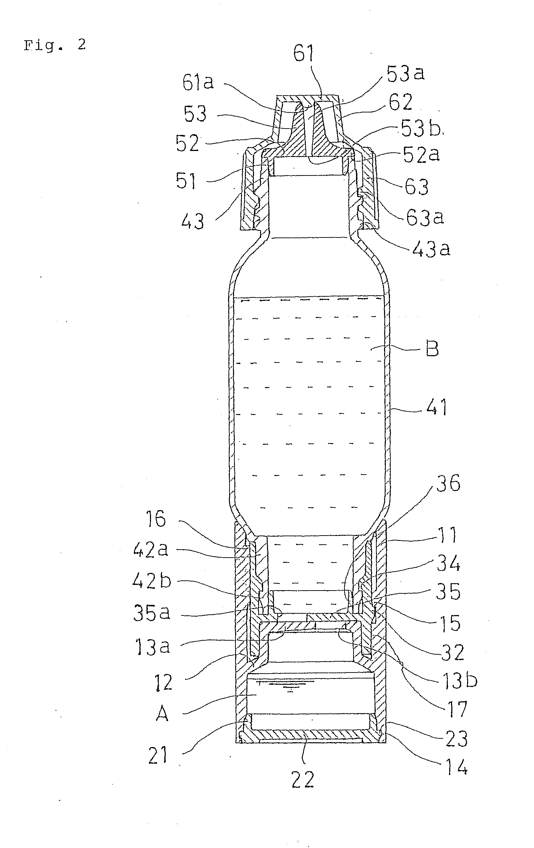

A movable body 1 is an injection-molded part made of synthetic resin. As shown in Figs. 1 to 3, the movable body 1 has a cylindrical tube section 11, an inner ledge 12 installed around so as to be inclined upward to a certain extent, and a ceiled short cylindrical inner tube 13 which is consecutively installed upright from the inner ledge 12, on an inner side in a circumferential direction from an inner surface of the tube section 11, and a first opening portion 13b is drilled in a ceiling plane 13a of the inner tube 13 and a locking groove line 14 is installed around in the inner surface of the lower end opening portion of the tube section 11. A bottom cover 2 is an inj ection-molded part made of synthetic resin. As shown in Figs. 1 and 2, a bottom plate 22 blocking up the lower end opening portion of the fit-into tube section 21 is provided at the lower end of a short cylindrical fit-into tube section 21, and an outer ledge 23 is formed so as to protrude outward in its radial direction from the peripheral edge of the bottom plate 22.

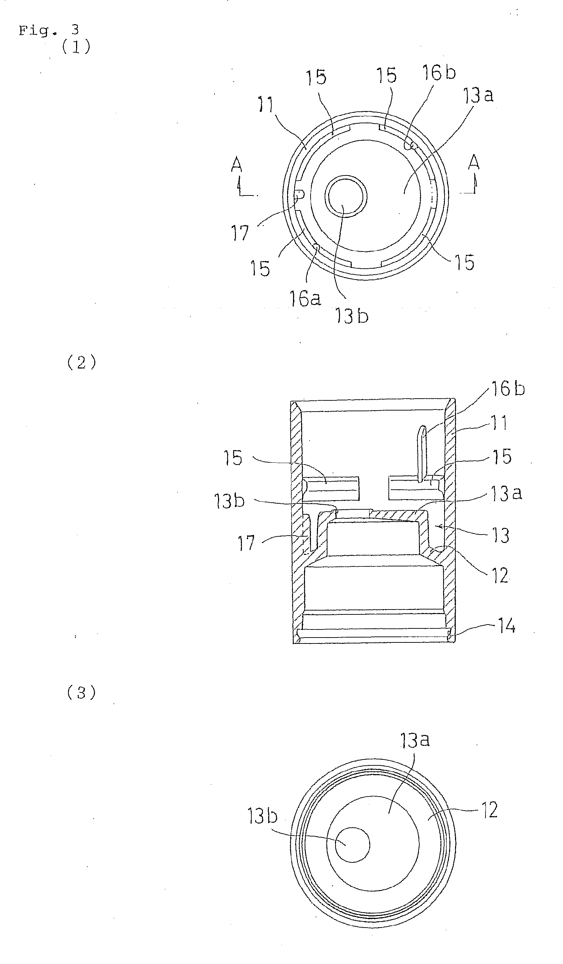

Then, the fit-into tube section 21 of the bottom cover 2 is tightly fit into the inner surface of the lower end opening portion of the tube section 11 of the movable body 1, and the outer ledge 23 of the bottom cover 2 is fit into the locking groove line 14 of the movable body 1, thereby making the bottom cover 2 fit into to be attached to the movable body 1 (refer to Fig. 2). As shown in Fig. 3, the inner ledge 12 and the inner tube 13 of the movable body 1 compose a partition section that partitions the internal space of the tube section 11 into two upper and lower chambers, and four locking protruding lines 15 are consecutively installed along its circumferential direction, and positioning protruding lines 16 (16a and 16b) are provided so as to protrude upward from substantially the central portion of the two locking protruding lines 15 facing each other on the inner surface of the tube section 11 composing the upper chamber above the partition section. Then, a first medicinal solution A is contained in the lower chamber under the partition section.

The adapter 3 is an inj ection-molded part made of synthetic resin. As shown in Figs. 1, 2, and 4, a hollow cylindrical fit-in tube section 31 is a primary member thereof, and the outer diameter of the fit-in tube section 31 is designed in size to have a diameter smaller to a certain extent than the inner diameter of the tube section 11 composing the upper chamber of the movable body 1. Two locking protruding lines 32 with a length which is approximately the half circle of the fit-in tube section 31 are consecutively installed so as to go around the fit-in tube section 31 on the outer surface of the fit-in tube section 31, and in the case in which the adapter 3 is tightly fit into the upper chamber of the movable body 1, these locking protruding lines 32 are locked with the locking protruding lines 15 of the movable body 1 in the axial line direction, thereby making the adapter 3 be assembled so as not to be allowed to be fit and removed and to be rotatable with respect to the movable body 1. In addition, at the time of assembling the adapter 3 to the movable body 1, it is recommended that groove portions 32a and 32b as gaps between the two locking protruding lines 32 and 32 be respectively fit into the positioning protruding lines 16a and 16b of the movable body 1, and the adapter 3 be pushed downward along the positioning protruding lines 16a and 16b.

On the other hand, a large number of longitudinal locking vertical lines 33 are provided so as to protrude at regular intervals along the circumferential direction starting from the upper end opening portion on the inner surface of the fit-in tube section 31, and locking protrusions 34 for locking are provided so as to protrude on the distal ends of the respective longitudinal locking vertical lines 33. Then, a partition wall surface 35 that partitions the internal space of the fit-in tube section 31 into two upper and lower chambers is provided on the lower side of the locking protrusions 34, and a short cylindrical airtight cap tube section 36 with a diameter shorter than that of the fit-in tube section 31 is provided upright on the peripheral edge of the partition wall surface 35, and a second opening portion 35a is drilled in the inner side of the airtight cap tube section 36 in the partition wall surface 35. The second opening portion 35a is disposed so as not to overlap the first opening portion 13b of the movable body 1 in a state in which the adapter 3 is assembled to the movable body 1, and is provided at a position at which the second opening portion 35a overlaps the first opening portion 13b when the movable body 1 is made to slide with respect to the adapter 3 to rotate around the center of axis. Then, as shown in Fig. 2, the adapter 3 and the movable body 1 are assembled in a state in which the partition wall surface 35 of the adapter 3 and the fit-in tube section 31 on the side lower than the partition wall surface 35 are fit at the outside of the inner tube 13 of the movable body 1 to be tightly attached in a liquid-tight manner.

A container main body 4 is a blow molded article made of synthetic resin. As shown in Figs. 1, 2, and 5, a second medicinal solution B is contained in the container main body 4, and a lower mouth tube section 42 is consecutively installed upright via its shoulder portion on the lower end of a cylindrical body section 41, and an upper mouth tube section 43 on which a screw portion 43a is carved is consecutively installed upright via the shoulder portion on the upper end of the body section 41. Four longitudinal locking vertical lines 42a are provided so as to protrude at regular intervals in the circumferential direction on the outer surface of the lower mouth tube section 42, and a peripheral protrusion line 42b is installed around on the outer surface of the lower end of the lower mouth tube section 42. In a state in which the lower mouth tube section 42 is tightly fit into the upper chamber surrounded by the upper surface of the partition wall surface 35 and the inner surface of the fit-in tube section 31 of the adapter 3, as shown in Fig. 2, the locking vertical lines 42a of the lower mouth tube section 42 are fit into between the locking vertical line 33 and the locking vertical line 33 on the inner surface of the fit-in tube section 31, thereby the container main body 4 and the adapter 3 are assembled so as not to be rotatable with respect to each other, and the peripheral protrusion line 42b of the lower mouth tube section 42 is locked in the axial line direction with the locking protrusion 34 on the inner surface of the fit-in tube section 31, thereby the container main body 4 and the adapter 3 are assembled so as not to be allowed to be fit and removed.

The nozzle 5 is an injection-molded part made of synthetic resin. As shown in Figs. 1 and 2, the nozzle 5 is provided with a short cylindrical airtight cap tube section 51 which is tightly fit into the inner surface of the upper end opening portion of the upper mouth tube section 43 of the container main body 4, a ceiling plane 52 having an outer ledge 52a that blocks up the upper end opening portion of the airtight cap tube section 51, and which is formed so as to protrude outward in a radial direction of the airtight cap tube section, and tightly touches the end face of the upper end opening portion of the upper mouth tube section 43 of the container main body 4 in a liquid-tight manner, and a nozzle section 53 which is formed so as to swell out on the central portion of the ceiling plane 52. An extracting orifice 53a is provided in the apical portion of the nozzle section 53, and the extracting orifice 53a is configured to have a depressed orifice wall in an inverted circular truncated cone shape from the apical portion of the nozzle section 53 up to the ceiling plane 52, and is configured to be communicated with the internal space of the airtight cap tube section 51 through an extracting hole 53b drilled in the lower end of the extracting orifice 53a of the nozzle section 53.

The cap 6 is an injection-molded part made of synthetic resin. As shown in Figs. 1 and 2, the cap 6 is composed of a ceiling plane 61, an upper tube body 62 in a tapered cylindrical shape whose diameter is extended gently downward from the peripheral edge of the ceiling plane 61, and a lower tube body 63 in a tapered cylindrical shape whose diameter is extended gently downward via the shoulder portion from the upper tube body 62. A screw portion 63a screwed in the screw portion 43a provided on the upper mouth tube section 43 of the container main body 4 is provided on the inner surface of the lower tube body 63, and a convex portion 61a which is tightly attached to the opening edge of the extracting orifice 53a is provided on the lower surface of the ceiling plane 61 in a liquid-tight manner. Further, an antislip knurl 64 is formed on the outer surface of the lower tube body 63.

That is, the before-use mixing container of the present invention is configured so that the bottom cover 2, the movable body 1, the adapter 3, the container main body 4, the nozzle 5, and the cap 6 are integrally assembled, and by use of the ceiling plane 13a of the inner tube 13 of the movable body 1 and the partition wall surface 35 of the adapter 3 as partition walls, the first medicinal solution A is contained in the lower chamber of the movable body 1 and the second medicinal solution B is contained in the container main body 4. Then, regarding the possibility of rotation between the above-described respective members with each other, the movable body 1 and the adapter 3 are assembled so as to be relatively rotatable with respect to each other, and the adapter 3 and the container main body 4 are assembled so as not to be relatively rotatable with respect to each other.

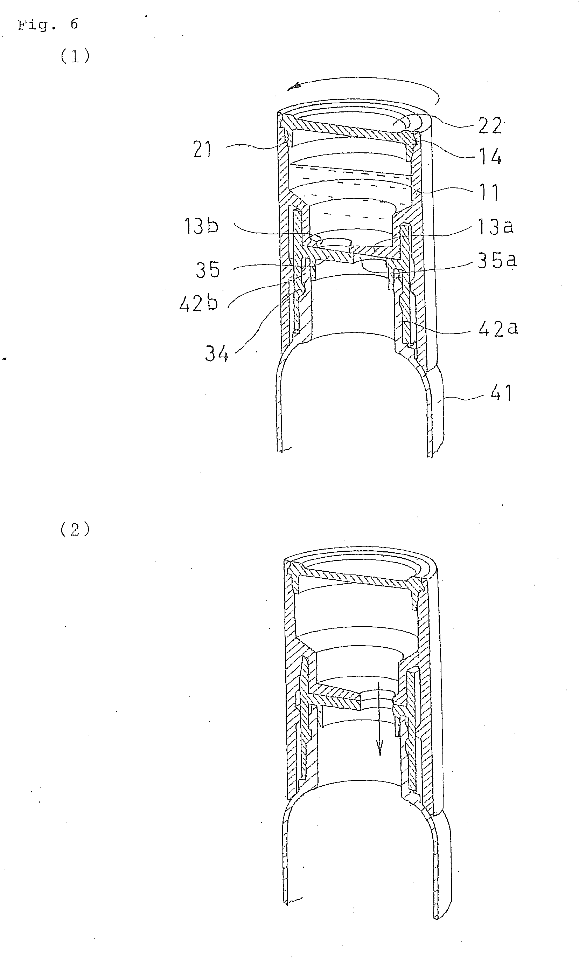

In use of the before-use mixing container of the present invention, a mixing operation for the first medicinal solution A and the second medicinal solution B, and an extraction operation for the mixed liquid are required. Regarding the former operation, as shown in Fig. 6A, the movable body 1 is rotated in a counterclockwise direction until the first opening portion 13b overlaps the second opening portion 35a in a state in which the container is turned upside down while grasping the container main body 4. Then, as shown in Fig. 6B, the first medicinal solution A contained in the movable body 1 falls into the container main body 4, thereby achieving mixing of the first medicinal solution A and the second medicinal solution B. Next, at the time of extracting the mixed liquid, provided that the container is returned to be in the original vertical direction, and the cap 6 is screwed out and the container is turned upside down, the mixed liquid passes through the extracting hole 53b to be extracted from the extracting orifice 53a. That is, it is possible to perform a mixing operation for the first medicinal solution A and the second medicinal solution B independently of an extraction operation in the present invention.

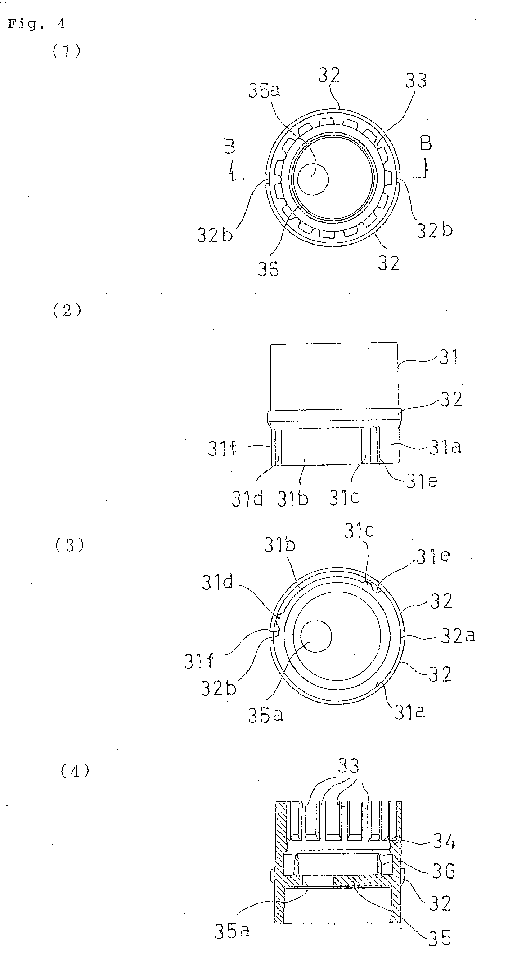

In the present embodiment, as shown in Fig. 4, a large diameter section 31a and a small diameter section 31b which are in substantially circular arc shapes in plan view are formed under the locking protruding lines 32 on the outer surface of the fit-in tube section 31 of the adapter 3, and in a state in which the fit-in tube section 31 of the adapter 3 is assembled to the upper chamber of the movable body 1, the large diameter section 31a and the small diameter section 31b of the fit-in tube section 31 are respectively spaced from the inner surface of the tube section 11 of the movable body 1 to generate gaps. Further, as shown in Figs. 2 and 3, a longitudinal vertical line 17 with a height exceeding a spaced distance between the inner surface of the tube section 11 and the large diameter section 31a, and with a height which does not exceed a spaced distance between the inner surface of the tube section 11 and small diameter section 31b is provided so as to protrude on the inner surface of the tube section 11 of the movable body 1, and this vertical line 17 faces the small diameter section 31b. With such a configuration, it is difficult to make the vertical line 17 on the inner surface of the tube section 11 of the movable body 1 climb over the large diameter section 31a rotate, and therefore, it is possible to keep the vertical line 17 to always face the small diameter section 31b.

Further, in the present embodiment, protrusion lines 31c and 31d are respectively provided in a longitudinal direction in the vicinity of one end side and in the vicinity of the other end side in the circumferential direction of the small diameter section 31b of the adapter 3, and locking groove lines 31e and 31f are respectively formed between the protrusion line 31c in the vicinity of the one end side in the circumferential direction of the small diameter section 31b and the other end side in the circumferential direction of the large diameter section 31a (the right end portion of the large diameter section in Fig. 4C), and between the protrusion line 31d in the vicinity of the other end side in the circumferential direction of the small diameter section 31b and the one end side in the circumferential direction of the large diameter section 31a (the left end portion of the large diameter section in Fig. 4C).

Then, in manufacturing of the container, at the time of making the fit-in tube section 31 of the adapter 3 be tightly fit into the upper chamber of the movable body 1, provided that the locking groove line 31e of the adapter 3 corresponding to a rotation starting position is fit into the vertical line 17 on the inner surface of the tube section 11 of the movable body 1 to make the adapter 3 be assembled to the movable body 1, in a mixing operation, when the movable body 1 is made to relatively rotate in the direction of an arrow in Fig. 6A with respect to the container main body 4, and the vertical line 17 climbs over the protrusion line 31c to pass through the small diameter section 31b, and climbs over the protrusion line 31d to be engaged with the locking groove line 31f, the movable body 1 is no longer able to be rotated, which terminates the rotation operation. That is, the locking groove lines 31e and 31f function as positioning respectively indicating the start and the end of rotation. Moreover, in the present embodiment, since the container is configured so that the first opening portion 13b and the second opening portion 35a are overlapped at the end of rotation, it is possible for a user to reliably perform a mixing operation.

The synthetic resins used for the above-described respective members are not particularly limited in type. For example, low-density polyethylene (LDPE), linear low-density polyethylene (LLDPE), high-density polyethylene (HDPE), polypropylene (PP), and the like may be appropriately combined for use. In particular, in order to improve the liquid-tight performance between the respective members, it is preferable that LDPE or LLDPE is used for the bottom cover 2 and HDPE is used for the movable body 1 in order to improve the liquid-tight performance between the bottom cover 2 and the movable body 1. Further, it is preferable that HDPE is used for the movable body 1 and PP is used for the adapter 3 in order to improve the liquid-tight performance between the movable body 1 and the adapter 3. As a result of study by the present inventors, a combination of synthetic resins in which the liquid-tight performance between the respective members is maximized is, from a comprehensive point of view, in the case in which LLDPE is used for the bottom cover, HDPE is used for the movable body, PP is used for the adapter, PP is used for the container main body, LDPE is used for the nozzle, and PP is used for the cap.

The embodiment of the present invention has been described above with reference to Figs. 1 to 6. However, modes of the respective members may be appropriately modified within a range which does not deviate from the scope of the present invention. For example, in the above-described embodiment, the ceiling plane 13a and the partition wall surface 35 are used as partition walls for the first medicinal solution A and the second medicinal solution B, and the first opening portion 13b and the second opening portion 35a are respectively provided therein. However, unless the opening portions provided in the upper and lower partition walls are not overlapped before mixing, to separate the first medicinal solution A and the second medicinal solution B, a plurality of opening portions in the upper and lower partition walls may be provided.

### [Embodiment]

The components of the container of the present invention were manufactured by use of synthetic resins shown in Table 1, test samples (A to D) in which the movable bodies and the container main bodies filled with medicinal solutions were assembled into products were prepared, and the liquid-tight performance between the respective components of these test samples were evaluated. The evaluations of the liquid-tight performance were carried out so that the test samples were overturned to be left in a state in which normal pressure was reduced by 70 kPa, and after the pressure was returned to normal pressure ten minutes later, it was visually confirmed whether or not there was liquid leakage from the gaps between the fitting portions of the respective components. In addition, the test samples having shapes according to those in Figs. 1 to 6, and in which its total height is 100 mm and the diameter of the tube section of the movable body is 22 mm were used. The results are shown in Table 2.

**[Table 1]**

| Test sample | Bottom cover | Movable body | Adapter | Container main body | Nozzle | Cap |
|---|---|---|---|---|---|---|
| A | LLDPE | LDPE | HDPE | PP | LDPE | PP |
| B | LDPE | HDPE | HDPE | | | |
| C | LDPE | LDPE | PP | | | |
| D | LLDPE | HDPE | PP | | | |

| | | | | | | |
|---|---|---|---|---|---|---|
| PP: Polypropylene LLDPE: Linear low-density polyethylene HDPE: High-density polyethylene LDPE: Low-density polyethylene | | | | | | |

**[Table 2]**

| Test sample | Bottom cover | Movable body | Adapter | Container main body | Nozzle |
|---|---|---|---|---|---|
| | Movable body | Adapter | Container main body | Nozzle | Cap |
| A | × | × | ○ | ○ | ○ |
| B | ○ | × | ○ | | |
| C | × | × | ○ | | |
| D | ○ | ▲ | ○ | | |

| | | | | | |
|---|---|---|---|---|---|
| ○: No leakage, ▲: Slight exudation, ×: Leakage | | | | | |

### [Industrial Applicability]

The present invention may have broad applicability as a before-use mixing container in which it is possible to perform a mixing operation for two types of medicinal solutions in its container independently of an uncovering operation for a cap or the like.

### [Description of Reference Numerals]

- 1: Movable body
- 3: Adapter
- 4: Container main body
- 11: Tube section
- 13: Inner tube
- 13a: Ceiling plane
- 13b: First opening portion
- 17: Vertical line
- 31: Fit-in tube section
- 31a: Large diameter section
- 31b: Small diameter section
- 35: Partition wall surface
- 35a: Second opening portion
- 42: Lower mouth tube section
- A: First medicinal solution
- B: Second medicinal solution

## Claims

1. A before-use mixing container comprising:
a movable body which has a bottomed cylindrical tube section, a ceiled cylindrical inner tube which is consecutively installed upright on an inner side in a circumferential direction from an inner surface of the tube section, that partitions an internal space of the tube section into two upper and lower chambers, and a first opening portion drilled in a ceiling plane of the inner tube, and in which a first medicinal solution is contained in the lower chamber;
an adapter which has a hollow cylindrical fit-in tube section which is tightly fit into the upper chamber of the movable body to be assembled so as to be rotatable with respect to the movable body, a partition wall surface which is provided on an inner surface of the fit-in tube section, and is tightly attached to the ceiling plane of the inner tube of the movable body in a state in which the partition wall is assembled to the movable body, and a second opening portion which is drilled in the partition wall surface so as not to overlap the first opening portion; and
a container main body which has a lower mouth tube section which is tightly fit into the upper chamber surrounded by an upper surface of the partition wall surface of the adapter and the inner surface of the fit-in tube section, to be assembled so as not to be rotatable with respect to the adapter, and in which a second medicinal solution is contained, wherein
when the movable body is relatively rotated with respect to the container main body, the first opening portion of the movable body and the second opening portion of the adapter are overlapped, and the container is capable of mixing the first medicinal solution and the second medicinal solution before use.

2. The before-use mixing container according to claim 1, wherein
the fit-in tube section of the adapter is provided with a large diameter section and a small diameter section having circular arc shapes in plan view, and in a state in which the fit-in tube section of the adapter is assembled to the upper chamber of the movable body, the large diameter section and the small diameter section of the fit-in tube section are respectively spaced from the inner surface of the tube section of the movable body to generate gaps, and
a vertical line with a height exceeding a spaced distance between the inner surface of the tube section and the large diameter section, and with a height which does not exceed a spaced distance between the inner surface of the tube section and the small diameter section is provided on the inner surface of the tube section of the movable body, and the longitudinal vertical line faces the small diameter section.

3. The before-use mixing container according to claim 2, wherein, when the movable body is relatively rotated with respect to the container main body and the vertical line provided on the inner surface of the tube section of the movable body reaches a boundary portion between the small diameter section and the large diameter section of the fit-in tube section, the first opening portion and the second opening portion are overlapped.
